# EUROPEAN PATENT SPECIFICATION

(11) **EP 1 138 351 B1**
(45) Date of publication and mention of the grant of the patent: **17.06.2009**
(21) Application number: 01302793.3
(22) Date of filing: 26.03.2001
(51) Int. Cl.: A63B 21/055, A61F 5/01, A63B 21/008, A63B 23/035

(54) **Excercise device**
Übungsgerät
Dispositif d'exercice

(30) Priority: 27.03.2000 US 535845
(43) Date of publication of application: 04.10.2001
(73) Proprietor: Pape, Leslie, Bedlington, Northumberland, NE22 5ER (GB)
(72) Inventor: Pape, Leslie, Bedlington, Northumberland, NE22 5ER (GB)
(74) Representative: Burrows, Anthony Gregory

(56) References cited:
- DE-A- 4 027 129
- DE-C- 661 680
- US-A- 445 726
- US-A- 4 171 802
- US-A- 5 190 511
- US-A- 5 358 468

## Description

This invention relates generally to exercise devices and, more particularly, to such a device worn by a user for flexing the user's upper body muscles, for example, the muscles in the shoulders, hips, upper arms, lower arms, upper legs, lower legs, ankles and wrists.

US-A-4,993,705 discloses an athletic device for the upper body, including a vest, and an elastically expandable strap fastened in place in an X-configuration across a back part of the vest and extended over the user's shoulders to terminate with two lower arm or wrist cuffs.

US-A-4,911,439 discloses a resilient exercise apparatus including a pair of loops made of a single length of elastic cord to form generally figure eight shape, and mounted over the shoulders and around the waist of the user, with a tubular handle mounted on each front and side sections of the cord.

US-A-3,162,441 discloses an exercise device including a frame for mounting on the back or chest of a user via upper and lower belts, three springs and a plurality of pulleys on the frame to accommodate a flexible cord with two stirrups.

GB-A-245,274 discloses an exercise device including a belt adapted to be secured around the waist or to be hung over the shoulders of the user, with elastic cords attached via springs to the belt, with hand grips at the ends thereof.

Exercise devices associated with waist belts and hand grips and/or wrist cuffs are shown and described in US-A-5,433,688; US-A-4,685,671; US-A-4, 540,173; US-A-4,441,707; US-A-3,999,752; US-A-2,035,010; US-A-1,432,013; and GB-A-20,463/1908.

DE-A-661680 discloses a rowing training machine including a base closed by a cover and containing a fluid. A shaft extends through the cover into a radial bearing in the base and at its upper end is swivelingly connected to a bar representing an oar. Keyed to the shaft is an encircling sleeve to which are fixed two diametrically opposite, radially outwardly extending windows obturated by respective, spring-biased, non-return, flap valves. Extending to the sleeve are two diametrically opposite, radially inwardly extending walls which have respective, slot-form, throttling openings therethrough containing adjustable throttling devices. Thus, the damping arrangement provides yielding resistance in only one sense about the shaft, which thereby simulates rowing.

Each of US-A-5,292,303; US-A-4,271,831 and US-A-5,409,449 discloses orthopaedic braces hinged at the knee joint of the wearer for free but adjustably limited knee movement. US-A-5,316,547 discloses an orthopaedic brace having medial and lateral knee hinges, with pneumatic pads for positioning against the body of a user.

US-A-5,358,468 discloses a knee rehabilitating and strengthening apparatus which includes upper and lower frame assemblies securable around a thigh and a shin of a user's leg above and below a knee joint, and a pair of separate torque unit assemblies disposed between the upper and lower frame assemblies at opposite lateral sides of and in alignment with the knee joint and pivotally interconnecting the upper and lower frame assemblies. The torque unit assemblies are operable adjustably to generate resistance to fluid flow in first and second opposite directions in response to respective pivotal movements of the upper and lower frame assemblies relative to one another in opposite directions upon flexion and extension of the wearer's leg. The apparatus also includes a fluid flow control arrangement connected, via flow branches external to the torque unit assemblies, with the torque unit assemblies and adjustable to preset independently a desired resistance to fluid flow in each of the first and second opposite directions through the fluid flow control arrangement and thereby independently adjust the amounts of work required to be exerted respectively in flexion and extension of the wearer's leg. A motion limiting arrangement is operable for adjustably presetting limits to pivotal movement of the upper and lower frame assemblies relative to one another and thereby respectively limit flexion and extension of the wearer's leg.

US-A-4,768,500 discloses an athletic knee protector with upper and lower leaf spring members interconnected by gear teeth pivotally engaged between a pair of plates.

According to the present invention, there is provided an exercise device for a person, said exercise device comprising first and second mounting straps for mounting on respective body members on opposite sides of a joint of the person, support members connected to the respective mounting straps, and yielding resistance means interconnecting the support members for exercising at least one of the two body members upon relative movement between said body members about said joint, wherein said resistance means is a damper which provides yielding resistance to said relative movement in respective opposite senses about said joint, said damper comprising a casing containing an oscillatory impeller and also a fluid for providing a predetermined damping torque in co-operation with oscillatory movement of the impeller by one of the body members, characterized in that said fluid is confined by said casing.

Owing to the invention, it is possible to provide an improved exercise device to be worn by a user to exercise body parts, such as muscles, adjacent his or her shoulder, hip, knee, ankle, elbow and/or wrist joints.

It is also possible to provide such exercise devices, which utilize upper and lower extensions with an intermediate damper for use on body parts straddling various body joints.

A particular advantage of the present invention is that the fluid is confined within the casing.

In order that the invention may be clearly and completely disclosed, reference will now be made, by way of example, to the accompanying drawings, in which:-
Figures 1 to 5 are side elevations of various, similar exercise device; adapted to being used on oppositely disposed body parts adjacent a knee joint, a hip joint, an ankle joint, a wrist joint, and an elbow joint, respectively;
Figure 6 is a front perspective view of a similar exercise devices adapted to being used on oppositely disposed body parts adjacent a shoulder joint;
Figure 7 is a rear perspective view of the exercise device of Figure 6;
Figure 8 is a perspective view of a lid of a rotary damper of the exercise devices of Figures 1 to 7;
Figure 9 is a perspective view of an impeller of the rotary damper;
Figure 10 is a perspective view of a base of the rotary damper;
Figure 11 is a perspective view of an impeller of an alternate rotary damper;
Figure 12 is a perspective view of a base of the alternate rotary damper;
Figures 13 to 18 are views corresponding to Figures 1 to 6, respectively, of various, alternate, similar, exercise devices which are adapted to being used on oppositely disposed body parts adjacent a knee joint, a hip joint, an ankle joint, a wrist joint, an elbow joint, a shoulder joint, respectively, but which are not according to the present invention; and
Figure 19 is a plan view of another alternate, rotary damper with a lid removed for sake of clarity.

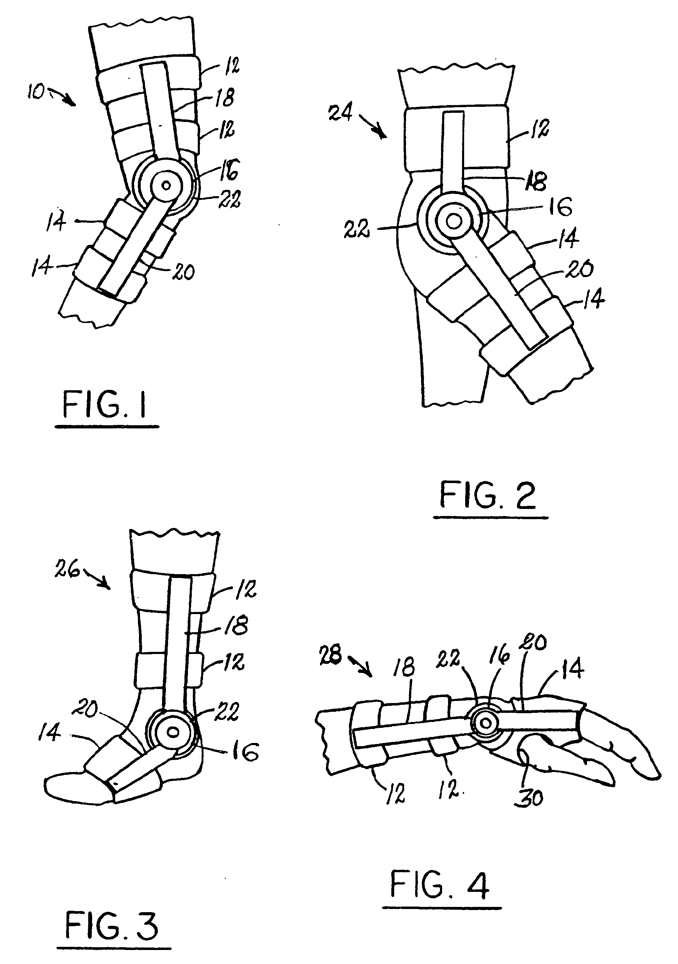

Referring now to the drawings in greater detail, Figure 1 illustrates an exercise device 10 for use on a user's leg, including a pair of spaced-apart upper mounting straps 12 (or a single mounting strap), secured around the leg above the knee joint by any suitable means, such as buckle, snap connector, or Velcro fastening (not shown); a pair of spaced-apart lower mounting straps 14 (or a single mounting strap) secured around the leg below the knee joint; a resistance mechanism in the form of a damper 16 adjacent the knee joint; an upper swing arm 18 attached to both upper straps 12 and to the damper 16; a lower swing arm 20 attached to both lower straps 14 and to the damper 16; and a cushion pad 22 secured to the inner surface of the damper 16 so as to be positioned between the damper 16 and the knee. As will be explained, the damper 16 serves to provide a yielding resistance to each repeated bending and straightening movement about the knee joint.

In Figure 2, an exercise device 24 includes the same elements as in Figure 1, but with a single upper strap 12 mounted around a user's waist and a pair of straps 14 (or only one strap) around an upper leg portion, so as to position the damper 16 and the cushion pad 22 adjacent a hip joint.

In Figure 3, an exercise device 26 likewise includes the same elements as in Figure 1, but mounted around a user's ankle, with a single mounting strap 14 around the user's foot, and with the damper 16 positioned adjacent an ankle joint.

In Figure 4, an exercise device 28 includes a pair of mounting straps 12 around the lower arm and a contoured strap 14 with a thumb hole 30 formed therein and mounted around a user's hand with the damper 16 and cushion pad 22 positioned adjacent the wrist joint.

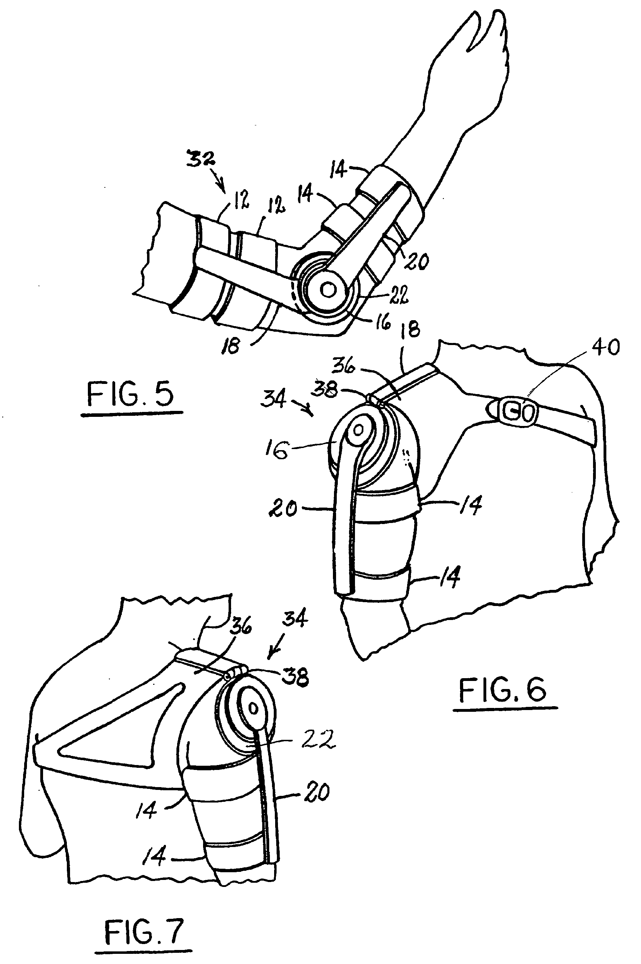

In Figure 5, an exercise device 32 is similar to the Figure 1 exercise device 10 but mounted relative to an elbow joint.

Figures 6 and 7 show an exercise device 34 adaptable to a shoulder joint. The device 34 includes a right- (or left-) hand shoulder strap 36 wrapped around the wearer's back, chest and opposite arm pit, connected together on the chest by any suitable means, such as a snap buckle 40. The upper swing arm 18 is secured to the shoulder strap 36 along the shoulder, with the outer end thereof connected by a suitable hinge 38 to an edge of the damper 16 so as to be turnable about an axis transverse to the joint axis. The lower swing arm 20 extends downwardly from an outer surface of the damper 16 to the two lower support straps 14 around the user's upper arm. The cushion pad 22 is between the damper 16 and the upper arm surface adjacent the shoulder.

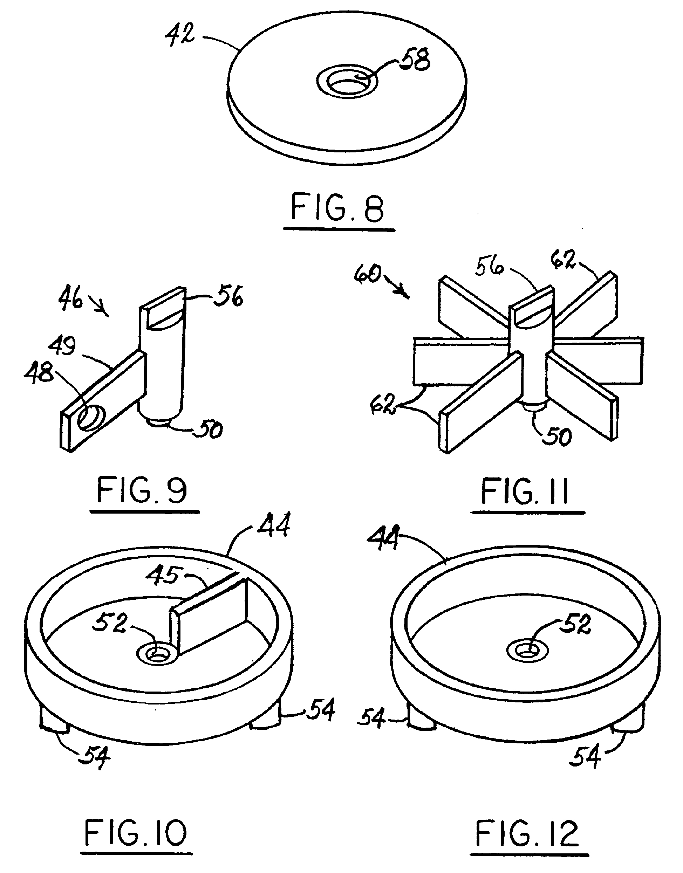

Referring now to Figures 8 to 10, there are shown three elements which form the damper 16. Specifically, the damper 16 includes a lid 42 for mounting on a base 44 including a radially extending inner wall 45, and enclosing an impeller 46 which has a throttling opening 48 of a predetermined throughflow cross-sectional area and formed in a radially outwardly extending wall in the form of a vane 49. The vane 49 is turnable in a selected viscous fluid (not shown) such as a silicone of a predetermined viscosity and, with the wall 45, divides the interior of the damper 16 into two chambers intercommunicating by way of the opening 48. The viscosity of the fluid and/or the opening size in the vane may be varied to increase or decrease the damping torque in co-operation with the back and forth flow of the fluid through the opening 48, the oscillation of the impeller 46 being limited only by the engagement of the vane 49 against a side of the wall 45. One formed end 50 of the impeller 46 is turnably mounted in an impeller bearing hole 52 formed in the bottom inner surface of the base 44. Location lugs 54 on an outer surface of the base 44 are secured in any suitable manner to one end of the upper or lower swing arm 18 or 20. A location connector 56 is formed at the other end of the impeller to extend through a sealed bearing hole 58 formed in the centre of the lid 42, for connection in any suitable manner to one end of the lower or upper swing arm 20 or 18 of each assembly.

As shown in Figures 11 and 12, an impeller 60 includes six vanes 62 in lieu of the perforated vane 49, and a base 44 with no inner wall 45. The vanes 62 and the inside diameter of the base 44 are each sized as desired to increase or decrease the rotary damping torque.

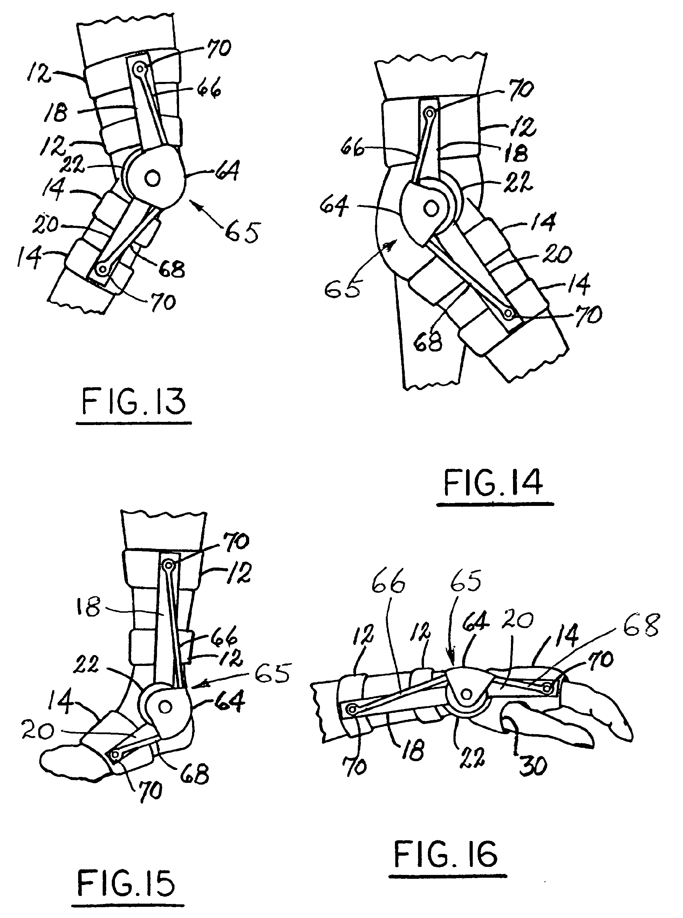

Figures 13, 14, 15, 16, 17 and 18 are comparable to Figures 1, 2, 3, 4, 5 and 6, respectively, except that the resistance mechanism in the form of the damper 16 is replaced with a resistance mechanism 65 comprised of a covered pulley 64 or other covered, arcuate- shaped element having an elastomeric member, such as a rubber band having oppositely disposed end portions 66 and 68, extending therearound and along respective upper and lower swing arms 18 and 20, and having its ends connected to the respective ends of the arms 18 and 20 by suitable studs 70. The pulley 64 may be a simple pulley wheel, with the rubber band 66, 68 looped around a portion thereof. Alternately, the rubber band 66, 68 may simply slide along a fixed, arcuate-shaped surface.

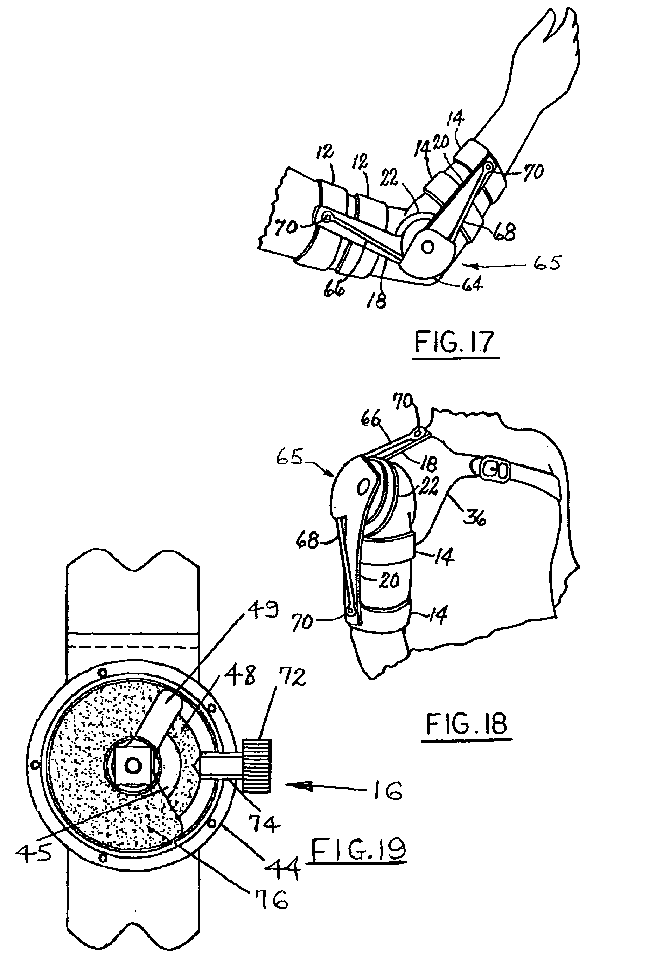

Referring to Figure 19, the damper 16 differs from that of Figures 9 and 10 in that the radially inwardly projecting wall 45 is formed with the throttling opening 48, that the vane 49 is imperforate, and that a set screw 72 engaged in an internally threaded bore 74 connected with the opening 48 is manually adjustable to change the throughflow cross-sectional area of the opening 48 in order to change the yielding resistance of the damper 16 provided by the viscous fluid 76.

In operation, relative movements of the shoulder and upper arm, upper and lower arm, lower arm and hand, upper and lower leg, or lower leg and foot, with the exercise device in place, serves to exercise these body members and their associated joints, so that virtually every muscle group adjacent all body joints can be exercised.

A particular advantage of the exercise devices described with reference to the drawings is that they are compact, without any significant projections away from the body parts, and so can be worn under ordinary clothing and used in everyday life. Thus, they are particularly convenient for use as everyday fitness exercise devices or physiotherapy exercise devices.

## Claims

1. An exercise device for a person, said exercise device comprising first and second mounting straps (12,14) for mounting on respective body members on opposite sides of a joint of the person, support members (18,20) connected to the respective mounting straps (12,14), and yielding resistance means (16;65) interconnecting the support members (18,20) for exercising at least one of the two body members upon relative movement between said body members about said joint, wherein said resistance means (16;65) is a damper (16) which provides yielding resistance to said relative movement in respective opposite senses about said joint, said damper (16) comprising a casing (42,44) containing an oscillatory impeller (46,60) and also a fluid (76) for providing a predetermined damping torque in co-operation with oscillatory movement of the impeller (46,60) by one of the body members, **characterized in that** said fluid (76) is confined by said casing (42,44).

2. A device according to claim 1, wherein said fluid is of selected viscosity.

3. A device according to claim 1 or 2, wherein said casing (42,44) comprises a base member (44) secured to one of said support members (18,20), and a cover (42), said impeller (46,60) being intermediate said base member (44) and said cover (42) and turnably mounted at one end (50) thereof, said fluid being confined between said base member (44) and said cover (42), said base member (44) including a radially inwardly projecting wall (45) and said impeller (46) comprising a radially outwardly projecting wall (49) which, with said inwardly projecting wall (45), divides the interior of said casing (42,44) into first and second sectoral chambers, and said damper (16) including, in said casing (42,44), a throttling opening (48) through which the first and second chambers inter-communicate, the oscillation of said outwardly projecting wall (49) forcing said fluid through said throttling opening (48).

4. A device according to claim 3, wherein said throttling opening (48) is formed through said outwardly projecting wall (49).

5. A device according to claim 3, wherein said throttling opening (48) is formed through said inwardly projecting wall (45).

6. A device according to claim 3, 4, or 5, wherein said damper (16) further includes a manually adjustable throttling element adjustable to set the throughflow cross-sectional area of said throttling opening (48).

7. A device according to claim 3, 4, or 5, wherein said impeller (60) includes a plurality of spaced, radially extending vanes (62) sized as required within said base member (44) so as to set the damping torque of said fluid during turning of said vanes (62).

8. A device according to any preceding claim, wherein each mounting strap (12,14) is connected to only one support member (18,20).

9. A device according to any preceding claim, wherein said joint is a shoulder joint and an upper one (18) of said support members (18,20) is connected to said resistance means (16;65) so as to be turnable about an axis transverse to the joint axis.

## Patentansprüche

1. Trainingsvorrichtung für eine Person, mit ersten und zweiten Befestigungsgurten (12,14) zur Befestigung an jeweiligen Körperteilen an entgegengesetzten Seiten eines Gelenks der Person, Stützteilen (18,20), die mit den jeweiligen Befestigungsgurten (12,14) verbunden sind, und mit einer nachgiebigen Widerstandsvorrichtung (16;65), welche die Stützteile (18,20) miteinander verbindet, um bei einer um das Gelenk herum durchgeführten Relativbewegung zwischen den beiden Körperteilen mindestens eines der beiden Körperteile zu trainieren, wobei die Widerstandsvorrichtung (16;65) eine Dämpfungsvorrichtung (16) ist, die der besagten Relativbewegung einen nachgiebigen Widerstand in jeweils gegenläufigen Richtungen um das Gelenk herum entgegensetzt, wobei die Dämpfungsvorrichtung (16) ein Gehäuse (42, 44) aufweist, das ein oszillierendes Flügelrad (46,60) und ferner ein Fluid (76) enthält, um in Zusammenwirkung mit einer durch eines der Körperteile bewirkten Schwingbewegung des Flügelrads (46,60) ein vorbestimmtes Dämpfungsdrehmoment zu erzeugen,
**dadurch gekennzeichnet, dass** das Fluid (76) in dem Gehäuse (42,44) eingeschlossen ist.

2. Vorrichtung nach Anspruch 1, bei der das Fluid eine gewählte Viskosität hat. '

3. Vorrichtung nach Anspruch 1 oder 2, bei der das Gehäuse (42,44) ein Basisteil (44), das an einem der Stützteile (18,20) befestigt ist, und einen Deckel (42) aufweist, wobei das Flügelrad (46,60) zwischen dem Basisteil (44) und dem Deckel (42) angeordnet und an einem Ende (50) drehbar befestigt ist, wobei das Fluid (76) zwischen dem Basisteil (44) und dem Deckel (42) eingeschlossen ist, wobei das Basisteil (44) eine radial nach innen abstehende Wand (45) aufweist und das Flügelrad (46) eine radial nach außen abstehende Wand (49) aufweist, die zusammen mit der nach innen abstehenden Wand (45) das Innere des Gehäuses (42,44) in erste und zweite Sektorkammern unterteilt, und die Dämpfungsvorrichtung (16) in dem Gehäuse (42,44) eine Drosselöffnung (48) enthält, durch die hindurch die ersten und zweiten Kammern miteinander kommunizieren, wobei die Schwingung der nach außen abstehenden Wand (49) das Fluid durch die Drosselöffnung (48) zwingt.

4. Vorrichtung nach Anspruch 3, bei der die Drosselöffnung (48) durch die nach außen abstehende Wand (49) hindurch ausgebildet ist.

5. Vorrichtung nach Anspruch 3, bei der die Drosselöffnung (48) durch die nach innen abstehende Wand (45) hindurch ausgebildet ist.

6. Vorrichtung nach Anspruch 3, 4 oder 5, bei der die Dämpfungsvorrichtung (16) ferner ein manuell einstellbares Drosselelement aufweist, das zur Bestimmung des Durchströmungs-Querschnittsbereichs der Drosselöffnung (48) einstellbar ist.

7. Vorrichtung nach Anspruch 3, 4 oder 5, bei der das Flügelrad (60) mehrere voneinander beabstandete, radial abstehende Flügel (62) aufweist, deren Abmessungen in dem Basisteil (44) derart gewählt sind, wie es zum Einstellen des Dämpfungsdrehmoments des Fluids während des Drehens der Flügel (62) erforderlich ist.

8. Vorrichtung nach einem der vorhergehenden Ansprüche, bei der jeder Befestigungsgurt (12,14) mit nur einem Stützteil (18,20) verbunden ist.

9. Vorrichtung nach einem der vorhergehenden Ansprüche, bei der das Gelenk ein Schultergelenk ist und ein oberes (18) der Stützteile (18, 20) derart mit der Widerstandsvorrichtung (16;65) verbunden ist, dass es um eine quer zu der Gelenkachse verlaufende Achse drehbar ist.

## Revendications

1. Dispositif d'exercice pour une personne, ledit dispositif d'exercice comprenant des première et seconde sangles de montage (12, 14) pour monter sur des éléments de corps respectifs sur les côtés opposés d'une articulation de la personne, des éléments de support (18, 20) raccordés aux sangles de montage (12, 14) respectives, et des moyens de résistance à l'élasticité (16 ; 65) interconnectant les éléments de support (18, 20) pour exercer au moins l'un des deux éléments de corps suite au mouvement relatif entre lesdits éléments de corps autour de ladite articulation,
dans lequel lesdits moyens de résistance (16 ; 65) est un amortisseur (16) qui fournit la résistance à l'élasticité audit mouvement relatif dans les sens opposés respectifs autour de ladite articulation, ledit amortisseur (16) comprenant un boîtier (42, 44) contenant un impulseur oscillant (46, 60) et également un fluide (76) pour fournir un couple d'amortissement prédéterminé en coopération avec le mouvement oscillant de l'impulseur (46, 60) par l'un des deux éléments de corps, **caractérisé en ce que** ledit fluide (76) est confiné par ledit boîtier (42, 44).

2. Dispositif selon la revendication 1, dans lequel ledit fluide a une viscosité sélectionnée.

3. Dispositif selon la revendication 1 ou 2, dans lequel ledit boîtier (42, 44) comprend un élément de base (44) fixé sur l'un desdits éléments de support (18, 20), et un couvercle (42), ledit impulseur (46, 60) étant situé entre ledit élément de base (44) et ledit couvercle (42) et monté de manière rotative au niveau de son extrémité (50), ledit fluide étant confiné entre ledit élément de base (44) et ledit couvercle (42), ledit élément de base (44) comprenant une paroi en saillie radialement vers l'intérieur (45) et ledit impulseur (46) comprenant une paroi en saillie radialement vers l'extérieur (49) qui, avec ladite paroi en saillie vers l'intérieur (45) divise l'intérieur dudit boîtier (42, 44) en première et seconde chambres sectorielles, ledit amortisseur (16) comprenant, dans ledit boîtier (42, 44), une ouverture d'étranglement (48) à travers laquelle les première et seconde chambres intercommuniquent, l'oscillation de ladite paroi en saillie vers l'extérieur (49) forçant ledit fluide à travers ladite ouverture d'étranglement (48).

4. Dispositif selon la revendication 3, dans lequel ladite ouverture d'étranglement (48) est formée à travers ladite paroi en saillie vers l'extérieur (49).

5. Dispositif selon la revendication 3, dans lequel ladite ouverture d'étranglement (48) est formée à travers la paroi en saillie vers l'intérieur (45).

6. Dispositif selon la revendication 3, 4 ou 5, dans lequel ledit amortisseur (16) comprend en outre un élément d'étranglement manuellement réglable, réglable pour régler la surface transversale d'écoulement de ladite ouverture d'étranglement (48).

7. Dispositif selon la revendication 3, 4 ou 5, dans lequel ledit impulseur (60) comprend une pluralité de pales (62) espacées s'étendant de manière radiale, dimensionnées si nécessaire dans ledit élément de base (44) afin de régler le couple d'amortissement dudit fluide pendant la rotation desdites pales (62).

8. Dispositif selon l'une quelconque des revendications précédentes, dans lequel chaque sangle de montage (12, 14) est raccordée uniquement à un élément de support (18, 20).

9. Dispositif selon l'une quelconque des revendications précédentes, dans lequel ladite articulation est une articulation d'épaule et un élément supérieur (18) desdits éléments de support (18, 20) est raccordé auxdits moyens de résistance (16 ; 65) afin de pouvoir tourner autour d'un axe transversal par rapport à l'axe de l'articulation.
